# EUROPEAN PATENT APPLICATION

(11) **EP 4 616 872 A1**
(43) Date of publication of application: **17.09.2025**
(21) Application number: 24382273.1
(22) Date of filing: 14.03.2024
(51) Int. Cl.: A61L 9/12, A01M 1/20

(54) **DEVICE FOR DIFFUSING VOLATILE SUBSTANCES AND METHOD FOR MANUFACTURING SAID DEVICE**

(71) Applicant: Zobele Holding SpA, 38100 Trento (IT)
(72) Inventor: SORDO, Walter, 38100 TRENTO (IT); TANG, Thomas, 38100 TRENTO (IT); DEFLORIAN, Stefano, 38100 TRENTO (IT)
(74) Representative: Herrero & Asociados, S.L.

(57) **Abstract**

The device for diffusing volatile substances comprises a container (1) for containing a liquid with the volatile substances comprising a rim (2); and a cover film (3) attached to the rim (2) of the container (1) that seals the container (1) before activation of the device, and that is partially separated from the container (1) after activation of the device, permitting the diffusion of the volatile substances, and an activation protrusion (4), so that pressing on said activation protrusion (4) activates the device, partially separating the cover film (3) from the rim (2) of the container (1).

It permits to provide a device in which its activation is very simple but, at the same time, it is difficult to be activated accidentally.

## Description

The present invention relates to a device for diffusing volatile substances and to a method for manufacturing said device.

### Background of the invention

Devices for diffusing volatile substances made from a container for liquid with the volatile substances made from flexible packaging are usually used. These containers consist in a thermoforming tray with a perimetral flange on which a thinner cover film is sealed in order to protect the liquid until the first use.

Usually, the cover film has to be peeled off by the user to get access to the liquid. In the last decades, it has been seen several solutions that deals about a semi-automatic aperture of this kind of containers.

These solutions are thought to be implemented in applications where the container is included inside a device or a housing. Therefore, the container is activated by a mechanical action of the device and no peeled off layer is removed from the container and shall be managed inside a device.

For example, some devices have been proposed where the cover film is perforated by some tooth or plunger in the device. However, with this kind of devices, special care as to be paid because the film, in order to be perforable by the teeth, need to be what use to be called a push-through film, that is the minimal thickness of a barrier layer and a minimal expression of a sealing layer. With this overall very thin thickness, it is quite challenging to find material that can support the chemical aggressivity of the substance contained.

Furthermore, the presence of the teeth in close proximity to the film, can lead to a preactivation, i.e., the teeth accidentally perforate the film, either during production process or during transportation.

For other examples, it is taken some advantage of the cover film weakness, either by a groove made in the film to reduce its thickness and thus to weaken film mechanical resistance, or by making the sealing between cover film and container weaker, or by reducing compatibility between the two layers, or by reducing welding width. Anyway, in this kind of solution, the container use to be highly deformed in order to generate high enough level of stress. This is quite common for manual activation, where user will bend or squeeze the container to activate it and then release it in order the container come back to undeformed shape.

Therefore, existing solutions are either based on perforation and thus very sensitive to preactivation or not adapted to be integrated inside a housing as they need high level of deformation of the container.

### Description of the invention

Therefore, an objective of the present invention is to provide a device for diffusing volatile substances and a method for its manufacturing in which its activation is very simple but, at the same time, it is difficult to be activated accidentally.

The device for diffusing volatile substances and the method of the invention solve the above-mentioned disadvantages and has other advantages which will be described below.

The device for diffusing volatile substances and the method according to the present invention are described in the independent claims, and the dependent claims include additional features that are optional.

In particular, the device for diffusing volatile substances comprises:
- a container for containing a liquid with the volatile substances comprising a rim;
- a cover film attached to the rim of the container that seals the container before activation of the device and that is partially separated from the container after activation of the device, permitting the diffusion of the volatile substances; and
- an activation protrusion, so that pressing on said activation protrusion activates the device, partially separating the cover film from the rim of the container.

Thanks to this feature, the activation is done just pressing the activation protrusion, with a tool or with a finger, so that the stress produced by said pressure, separates partially the cover film from the rim of the container.

Preferably, the activation protrusion can comprise a flange that protrudes externally from the container, so that the activation protrusion can be pressed easily also pressing on said flange.

Furthermore, the rim of the container is preferably placed coplanar with a top surface of the activation protrusion, and the cover film can be attached to the top surface of the activation protrusion.

According to a preferred embodiment, the activation protrusion comprises conical side walls or walls defining a conical shape, which can include a plurality of concentric recesses, defining a bellows.

Furthermore, the activation protrusion can placed in a portion of the container that has a depth less than the rest of the container.

According to a possible embodiment, the device can also comprise a cavity separated from the rest of the container by an intermediate area, and an additional activation protrusion placed in said cavity.

Also in this case, the additional activation protrusion comprises a flange that protrudes externally from the container, the rim of the container can be placed coplanar with a top surface of the additional activation protrusion, and the cover film can be attached to the top surface of the additional activation protrusion.

According to a possible embodiment, the additional activation protrusion can comprise conical side walls or walls defining a conical shape with a plurality of concentric recesses, defining a bellows.

Preferably, the intermediate area has a width that decreases towards its center.

According to a second aspect, the method for manufacturing a device for diffusing volatile substances as describe previously comprises the steps of:
- thermoforming the activation protrusion at a height higher than the rest of the container; and
- attaching the cover film to the container, compressing the activation protrusion until it moves back to the level of the rim of the container.

### Brief description of the drawings

For a better understanding of what has been explained above, some drawings are included in which, schematically and only by way of a non-limiting example, a practical case of embodiment is represented.
Figure 1 is a perspective view from above of the device for diffusing volatile substances according to the present invention, with the cover film separated from the rest of the device for view the internal part of the container;
Figure 2 is a perspective view from below of the device for diffusing volatile substances shown in Figure 1;
Figure 3 is a cross-sectional view of the activation protrusion of the device for diffusing volatile substances according to the present invention.

### Description of a preferred embodiment

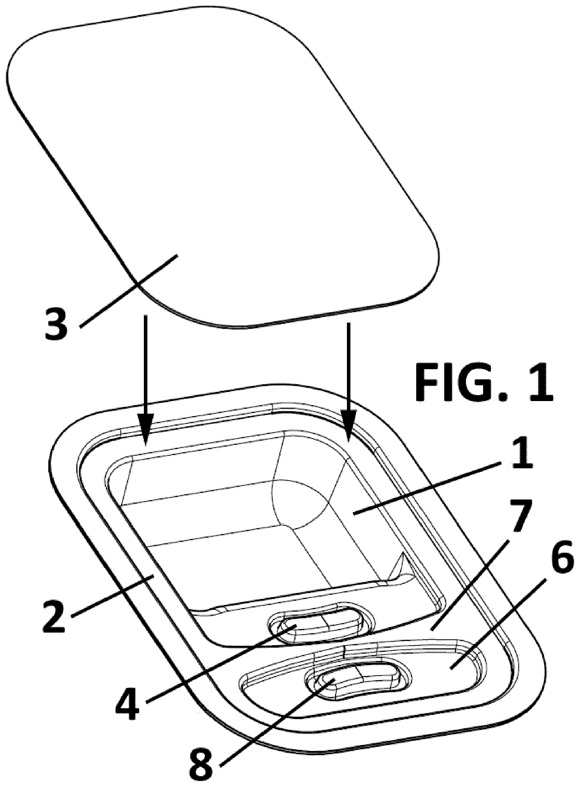

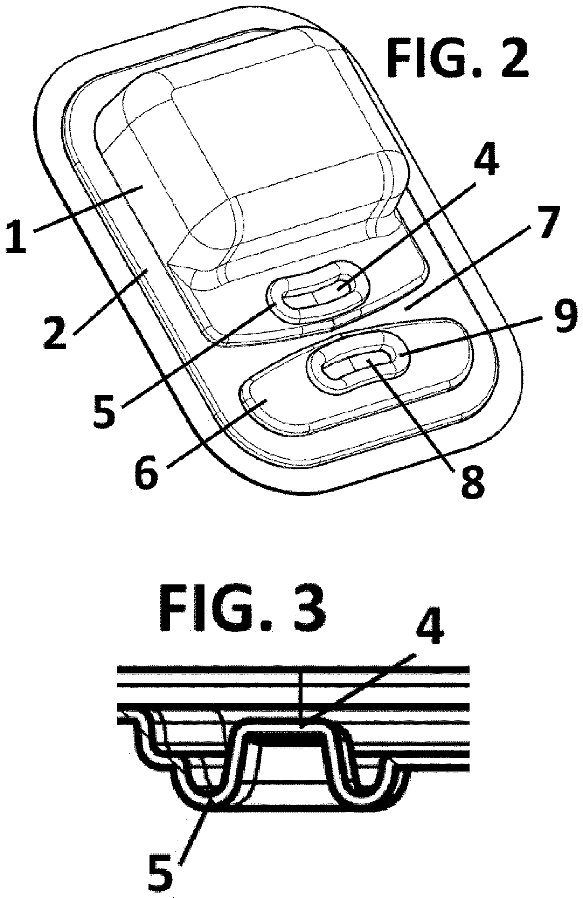

Figures 1 and 2 show an embodiment of the device for diffusing volatile substances according to the present invention.

The device comprises a container (1) for containing a liquid with the volatile substances comprising a rim (2), and a cover film (3) attached, e.g., by welding, to the rim (2) of the container (1) that seals the container (1) before activation of the device, and that is partially separated from the container (1) after activation of the device, permitting the diffusion of the volatile substances.

Furthermore, the device comprises an activation protrusion (4), placed in the central part (2) of the container (1) in proximity of the rim, so that pressing on said activation protrusion (4) activates the device, partially separating the cover film (3) from the rim (2) of the container (1).

When pressed, the activation protrusion generates a stress on an area of rim (2) in contact with the cover film (3), that pushes the cover film (3) outwardly. The more the cover film (3) is pushed outwardly, the higher the stress on the interface between the cover film (3) and the rim (2) will be, leading the aperture of the container (1) by separation.

Furthermore, the activation protrusion (4) comprises preferably a flange (5) that protrudes externally from the container (1), and a pressure can be applied also on said flange (5) for pressing the activation protrusion (4).

According to the preferred embodiment, the rim (2) of the container (1) is placed coplanar with a top surface of the activation protrusion (4), and the cover film (3) is attached to the top surface of the activation protrusion (4).

This feature has the effect that the cover film (3) has lower freedom of movement and all deformation of the activation protrusion (4) towards the cover film (3) will be used to generate stress at the interface between the rim (2) and the cover film (3).

For pressing the activation protrusion (4) and/or the flange (5) a tool or a finger can be used, producing the enough stress for activating the device.

It must be pointed out that the activation protrusion (4) is made by thermoforming at a height higher than the rest of the container (1), in particular, a height higher than the rim (2) of the container (1). Then, when the cover film (3) is attached to the container (1), the activation protrusion (4) is compressed until it moves back to the level of the rim (2) of the container (1). This feature permits that the activation protrusion (4) can be deformed easier towards the cover film (3) when it is pressed.

However, once activated, the activation protrusion (4) can remain with a remaining deformation, i.e., not returning in its original position, to let the liquid flow from the container (1) with no impediments.

During that operation, the cover film (3) can be also attached to the activation protrusion (4). This way, an easier deformation of the protuberance when it is pushed forward can be obtained.

Having this higher depth of the activation protrusion (4), on one side it is obtained an activation protrusion (4) which preform is naturally deeper than the depth of the container (1) and on the other side the higher deformation during thermoforming will lead to lower wall thickness of the container (1).

As shown in Figure 1, the activation protrusion (4) can be placed in a portion of the container (1) that has a depth less than the rest of the container (1). This way, the design of the activation protrusion (4) and the area around it is not linked to the capacity of the container (1) and it can be adjusted in order to allow easy movement of the activation protrusion (4) in the direction of the cover film (3).

As shown in Figure 3, preferably the activation protrusion (4) comprises conical side walls, or a biased profile, and the activation protrusion (4) can also comprise side walls with a plurality of concentric recesses forming a bellows. This permits an easier movement of the activation protrusion (4).

Preferably, the shape of the activation protrusion (4) complementary with the shape of the rim (2).

Even though it is not necessary, the embodiment of the device shown in Figures 1 and 2 comprises a cavity (6) separated from the rest of the container (1) by an intermediate area (7), and an additional activation protrusion (8) placed in said cavity (6).

This additional activation protrusion (8) is preferably identical to the activation protrusion (4) described previously.

As shown in these figures, said intermediate area (7) has preferably a width that decreases towards its center.

For example, the cavity (6) can be an open container in direct contact with the surrounding atmosphere, and when the device is activated, part of the liquid inside the container (1) passes to the cavity (6), where evaporates passively.

Furthermore, in this embodiment, both protrusions (4, 8) can be pressed, and the interface between the rim (2) and the cover film (3) is subjected to stress from both sides.

It must be noted that if the cavity (6) is present, the protrusion (4) can be placed only in the container (1), or only in the cavity (6), or both protrusions (4, 8) can be placed one in the container (1) and one in the cavity (6), as shown in the drawings.

Alternatively, the rims around the container and/or the cavity are surrounded by an outer rim on which a permeable membrane is fixed, defining a space where the volatile liquid is transferred after device activation and allowing its evaporation through the membrane.

Although reference has been made to a specific embodiment of the invention, it is obvious to a person skilled in the art that the device for diffusing volatile substances described and method are susceptible to numerous variations and modifications, and that all the details mentioned can be replaced by technically equivalent ones, without departing from the scope of protection defined by the appended claims.

## Claims

1. Device for diffusing volatile substances, comprising:
- a container (1) for containing a liquid with the volatile substances comprising a rim (2); and
- a cover film (3) attached to the rim (2) of the container (1) that seals the container (1) before activation of the device, and that is partially separated from the container (1) after activation of the device, permitting the diffusion of the volatile substances, **characterized in that** the device comprises an activation protrusion (4), so that pressing on said activation protrusion (4) activates the device, partially separating the cover film (3) from the rim (2) of the container (1).

2. Device for diffusing volatile substances according to claim 1, wherein the activation protrusion (4) comprises a flange (5) that protrudes externally from the container (1).

3. Device for diffusing volatile substances according to claim 1 or claim 2, wherein the rim (2) of the container (1) is placed coplanar with a top surface of the activation protrusion (4).

4. Device for diffusing volatile substances according to claim 3, wherein the cover film (3) is attached to the top surface of the activation protrusion (4).

5. Device for diffusing volatile substances according to any one of the previous claims, wherein the activation protrusion (4) comprises conical side walls.

6. Device for diffusing volatile substances according to any one of the previous claims, wherein the activation protrusion (4) comprises side walls with a plurality of concentric lines.

7. Device for diffusing volatile substances according to any one of the previous claims, wherein the activation protrusion (4) is placed in a portion of the container (1) that has a depth less than the rest of the container (1).

8. Device for diffusing volatile substances according to any one of the previous claims, wherein the device also comprises a cavity (6) separated from the rest of the container (1) by an intermediate area (7), and an additional activation protrusion (8) placed in said cavity (6).

9. Device for diffusing volatile substances according to claim 8, wherein the additional activation protrusion (8) comprises a flange (9) that protrudes externally from the container (1).

10. Device for diffusing volatile substances according to claim 8 or claim 9, wherein the rim (2) of the container (1) is placed coplanar with a top surface of the additional activation protrusion (8).

11. Device for diffusing volatile substances according to claim 10, wherein the cover film (3) is attached to the top surface of the additional activation protrusion (8).

12. Device for diffusing volatile substances according to any one of claims 8-11, wherein the additional activation protrusion (8) comprises conical side walls.

13. Device for diffusing volatile substances according to any one of claims 8-12, wherein the additional activation protrusion (8) comprises side walls with a plurality of concentric recesses.

14. Device for diffusing volatile substances according to claim 8, wherein the intermediate area (7) has a width that decreases towards its center.

15. Method for manufacturing a device for diffusing volatile substances according to anyone of the previous claims, **characterized in that** the method comprises the steps of:
- thermoforming the activation protrusion (4) at a height higher than the rest of the container (1); and
- attaching the cover film (3) to the container (1), compressing the activation protrusion (4) until it moves back to the level of the rim (2) of the container (1).
